# EUROPÄISCHE PATENTANMELDUNG

(11) **EP 0 884 344 A2**
(43) Veröffentlichungstag der Anmeldung: **16.12.1998**
(21) Anmeldenummer: 98109730.6
(22) Anmeldetag: 28.05.1998
(51) Int. Cl.: C08G 73/10, C11D 3/37, A61K 7/06, A61K 7/16, A61K 7/48

(54) **Milde tensidhaltige Zubereitungen mit copolymeren Polyasparaginsäurederivaten für kosmetische oder Reinigungszwecke**

(30) Priorität: 11.06.1997 DE 19724589
(71) Anmelder: Th. Goldschmidt AG, 45127 Essen (DE)
(72) Erfinder: Grüning, Burghard, Dr., 45134 Essen (DE); Rau, Harald, 45138 Essen (DE); Simpelkamp, Jörg, Dr., 45130 Essen (DE); Weitemeyer, Christian, Dr., 45134 Essen (DE)

(57) **Zusammenfassung**

Diese Erfindung beschreibt milde Tensidzübereitungen für Reinigungszwecke sowie deren Einsatz in der Kosmetik, welche ein oder mehrere überwiegend lineare oberflächenaktive Polyasparaginsäurederivate, ein oder mehrere weitere Tenside aus der Gruppe der nichtionischen, kationischen, zwitterionischen und amphoteren Tenside sowie deren Mischungen oder wenigstens ein anionisches Tensid in Kombination mit wenigstens einem anionischen, kationischen, nichtionischen, zwitterionischen und/oder amphoteren Tensid neben übliche Hilfs- und Zusatzstoffe enthalten.

## Beschreibung

Diese Erfindung beschreibt milde Tensidzubereitungen für Reinigungszwecke sowie deren Einsatz in der Kosmetik, welche ein oder mehrere überwiegend lineare oberflächenaktive Polyasparaginsäurederivate, ein oder mehrere weitere Tenside aus der Gruppe der nichtionischen, kationischen, zwitterionischen und amphoteren Tenside sowie deren Mischungen oder wenigstens ein anionisches Tensid in Kombination mit wenigstens einem anionischen, kationischen, nichtionischen, zwitterionischen und/oder amphoteren Tensid neben üblichen Hilfs- und Zusatzstoffe enthalten.

Polyaminosäurederivate, insbesondere Polyasparaginsäure, haben in jüngster Zeit aufgrund ihrer Eigenschaften besondere Aufmerksamkeit gefunden. Es werden u.a. Anwendungen als biologisch abbaubare Komplexierungsmittel, Enthärter und Waschmittel-Builder vorgeschlagen. Polyasparaginsäure wird i.A. durch alkalische Hydrolyse der unmittelbaren Synthesevorstufe Polysuccinimid (PSI, Anhydropolyasparaginsäure), dem cyclischen Imid der Polyasparaginsäure gewonnen. PSI kann beispielsweise nach EP 0 578 449 A, WO 92/14753, EP 0 612 784 A oder DE 43 00 020 A aus Asparaginsäure hergestellt werden oder ist beispielsweise nach DE 36 26 672 A, EP 0 650 995 A, EP 0 693 516 A oder US 5 219 952 A aus Maleinsäurederivaten und Ammoniak zugänglich. Derartige Verbindungen sind mehr oder weniger stark verzweigt, wobei insbesondere höherverzeigte Polyasparaginsäuren eine schlechtere biologische Abbaubarkeit besitzen (M. Freeman et al., CESIO Surfactants Symposium Proceedings Barcelona 1996, 250-263).

Für diese üblichen Polyasparaginsäuren werden u.a. Anwendungen als Inkrustationsinhibitor, Builder in Waschmitteln, Düngemitteladditiv und Hilfsstoff in der Gerberei vorgeschlagen.

Die von verschiedenen Arbeitsgruppen beschriebene Umsetzung von Polysuccinimid mit Aminen führt zu Polyasparaginsäureamiden (Kovacs et al., J. Med. Chem. 1967, 10, 904-7; Neuse, Angew. Makromol. Chem. 1991, 192, 35-50). Neri et al. führen die Ringöffnung von PSI mit Ethanolamin durch und erhalten Hydroxyethylaspartamide (J. Med. Chem. 1973, 16, 893-897, Macromol. Synth. 1982, 8, 25-29). DE 37 00 128 A und EP 0 458 079 A beschreiben die nachfolgende Veresterung derartiger Hydroxyethylderivate mit Carbonsäurederivaten und die Verwendung der Produkte in Ultraschallkontrastmitteln und in Wirkstoffdepotzubereitungen.

DE 195 24 097 A1 beschreibt Tensidzubereitungen aus Alkyl- oder Alkenylpolyglucosiden und/oder Fettsäure-N-alkylpolyhydroxyalkylamiden im Gemisch mit hydrophobierten Oligopeptiden, beispielsweise Fettsäure-Eiweiß-Kondensationsprodukten oder Umsetzungsprodukten von Polyasparaginsäure mit Fettalkoholen oder -aminen mit 6-22 C-Atomen. Die übliche Veresterung von Polyasparaginsäure mit Fettalkoholen nach den dort nicht in nachvollziehbarer Weise beschriebenen Verfahren weist jedoch gravierende Nachteile auf, da ohne den unter ökonomischen und ökologischen Gesichtspunkten nachteiligen Einsatz grosser Mengen an oft schwierig zu entfernenden organischen Lösungsmitteln nur inhomogene Produkte mit geringem Umsetzungsgrad erhalten werden, die ein kommerzielle Verwertung mit einem vertretbaren Aufwand nicht zuläßt. Darüber hinaus weisen diese aus Polyasparaginsäure hergestellten Produkte zwangsläufig Verzweigungen auf, mit den bekannten negativen Auswirkungen auf die biologische Abbaubarkeit.

Dagegen sind homogene, copolymere, hydrophob modifizierte Polyasparaginsäureester auf Basis von Maleinsäuremonoestern und Ammoniak leicht zugänglich, wie aus den noch nicht offengelegten DE 195 45 678 oder EP 96 118 806.7 hervorgeht, auf die expressis verbis Bezug genommen wird. Diese Produkte besitzen einen überwiegend linearen Aufbau, wie sich aus der Lehre zum Mechanismus des Polyasparaginsäureaufbaus aus Maleinsäurederivaten ergibt, beispielsweise aus der DE 43 00 020 A.

Die Mildheit und dermatologische Verträglichkeit von tensidischen Zübereitungen spielt in verschiedenen Anwendungsgebieten, insbesondere aber auf dem kosmetischen Sektor, eine wichtige Rolle. Dabei sind naturnahe Grundstrukturen der verwendeten Tenside von Vorteil, wie sie beispielsweise die in den letzten Jahren in den Blickpunkt des Interesses getretenen Alkylpolyglucoside besitzen. So beschreibt DE 43 19 699 A besonders milde Tensidmischungen aus Alkyloligoglucosiden. Aus wirtschaftlichen und anwendungstechnischen Gründen kann jedoch in tensidischen Zubereitungen meist nicht auf den Einsatz von anionischen Tensiden wie z.B. Alkylsulfaten oder Alkylethersulfaten mit hohem Reizpotential verzichtet werden. Die dermatologischen Eigenschaften derartiger Zubereitungen lassen sich oft durch den Einsatz von Sekundärtensiden verbessern wie beispielsweise Betainen (beschrieben z.B. in DE 33 05 197 A, DE 30 11 549 A oder von Dominguez et al. in: Goldschmidt informiert 1981, 55, 10) oder Alkylpolyglucosiden (wie in WO 93/23512 oder DE 42 34 487 A), wobei allerdings oft Einschränkungen in den Anwendungseigenschaften in Kauf genommen werden müssen.

Es ist daher die Aufgabe der Erfindung, außerordentlich milde und reizarme tensidische Zubereitungen zur Verfügung zu stellen, die dem Anspruch von hoher Hautverträglichkeit und guten Anwendungseigenschaften genügen.

Die vorgenannte Aufgabe wird erfindungsgemäß gelöst durch tensidische Zubereitungen für Reinigungsmittel und/oder kosmetische Mittel, enthaltend ein oder mehrere überwiegend geradkettige Polyasparaginsäurederivate und ein oder mehrere weitere Tenside aus der Gruppe der anionischen, kationischen, nichtionischen, amphoteren und zwitterionischen Tenside sowie deren Mischungen neben üblichen Hilfs- und Zusatzstoffen.

Überraschend zeigte sich, daß überwiegend lineare Polyasparaginsäurederivate mit einer naturnahen Polyaminosäurestruktur sich beim Einsatz auch in bereits als mild geltenden Tensidzubereitungen mit einem oder mehreren weiteren Tensiden durch eine deutliche Verbesserung der Mildheit auszeichnen, wobei die gewünschten Anwendungseigenschaften, z.B. Schaumeigenschaften nicht beeinträchtigt oder sogar günstig beeinflußt werden.

Erfindungsgemäß eingesetzte Polyasparaginsäurederivate sind beispielsweise die durch Umsetzung von Maleinsäuremonoestern mit Ammoniak zugänglichen copolymeren Polyasparaginsäureester, welche durch eine Polyasparaginsäurestruktur gekennzeichnet sind, bei der die Seitenketten partiell als freie Carbonsäure- bzw. Carboxylatgruppen vorliegen und partiell mit einem oder mehreren Alkoholen mit 1-30 C-Atomen, darunter mindestens ein langkettiger Fettalkohol, vorzugsweise mit 6-24 C-Atomen, oder dessen Derivaten verestert sind.

Die eingesetzten, von Polyasparaginsäure abgeleiteten Copolymeren bestehen zu wenigstens 75 %, insbesondere Mol.-%, der vorhandenen Einheiten aus Struktureinheiten der allgemeinen Formeln (I) und (II) , wobei A ein trifunktionelles Kohlenwasserstoffradikal mit 2 C-Atomen der Struktur (A1) oder (A2) ist, 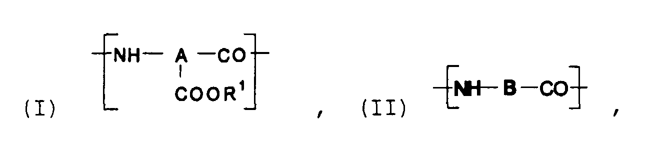 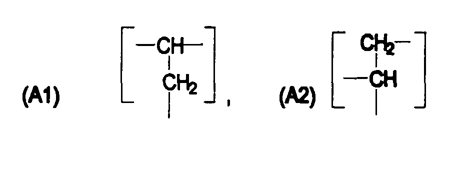 worin R1 die Bedeutung von R2, R3 und R4 haben kann, wobei
- R²: für ein oder mehrere Reste aus der Gruppe der Alkali-, Erdalkalimetalle, Wasserstoff oder Ammonium, [NR⁵R⁶R⁷R⁸]⁺ sind, worin R⁵ bis R⁸ unabhängig voneinander Wasserstoff, Alkyl oder Hydroxyalkyl mit 1 bis 30 C-Atomen ist,
- R³: für gleiche oder verschiedene, geradkettige oder verzweigte, gesättigte oder ungesättigte Alkylreste R⁹ mit 6 bis 24 C-Atomen oder Radikale der Struktur -X-R⁹, wobei X eine Oligo- oder Polyoxyalkylenkette mit 1 bis 100 Oxyalkyleneinheiten ist, und
- R4: für gleiche oder verschiedene, geradkettige oder verzweigte, gesättigte oder ungesättigte Alkylreste mit 1 bis 5 C-Atomen steht,
und wenigstens jeweils ein Rest R1 die Bedeutung von R2 und wenigstens ein Rest R1 die von R3 annehmen muß und
die Einheiten [-NH-B-CO] Bausteine aus der Gruppe der proteinogenen oder nicht proteinogenen Aminosäuren sind und zu nicht mehr als 20 Gew.-% enthalten sind.

Alle gegebenen Angaben zur Zusammensetzung der polymeren Produkte beziehen sich vorzugsweise wie üblich auf die mittlere Zusammensetzung der Polymerketten.

Als Aminosäurebausteine [-NH-B-CO] aus der Gruppe der proteinogenen Aminosäuren kommen z. B. Glutamin, Asparagin, Lysin, Alanin, Glycin, Tyrosin, Tryptophan, Serin und Cystein sowie deren Derivate in Frage; nicht proteinogene Aminosäuren können beispielsweise β-Alanin, ω-Amino-1-alkansäuren etc. sein.

Erfindungsgemäß bevorzugt sind Verbindungen, bei denen wenigstens eine freie Carboxylatgruppe (R¹ = H, Metall, Ammonium) vorhanden ist, wenigstens ein Rest R³ gleiche oder verschiedene Radikale der Struktur R⁹-X- umfaßt, wobei R⁹ aus der Gruppe der geradkettigen oder verzweigten, gesättigten oder ungesättigten Alkylreste mit 6 bis 24 C-Atomen stammt (z. B. verzweigte oder lineare Octyl-, Decyl-, Dodecyl-, Tetradecyl-, Hexadecyl-, Octadecyl-, Docosylreste, auch ungesättigte und mehrfach ungesättigte Spezies wie z. B. Oleyl) und X eine Polyoxyalkylenkette von 0 bis 100 Alkylenglykoleinheiten, vorzugsweise abgeleitet von Ethylenoxid, Propylenoxid oder Gemischen daraus und ggf. ein Rest R⁴ aus der Gruppe der geradkettigen oder verzweigten, gesättigten oder ungesättigten Alkylreste mit 1 bis 5 C-Atomen stammt (z. B. Methyl, Ethyl, n-Propyl, i-Propyl, n-Butyl, i-Butyl, s-Butyl, n-Pentyl). Eine weiter bevorzugte Form der Copolymeren enthält Alkylreste R⁹ mit 6 bis 18 C-Atomen ohne Alkylenglykolspacer (Alkylenglykolkettenlänge 0) sowie ggf. geringe Mengen von Alkylresten mit 1-4 C-Atomen.

Aufgrund der guten Anwendungseigenschaften und besseren biologischen Abbaubarkeit von Derivaten mit linearem Polyasparaginsäuregerüst ist ein linearer Aufbau der Ester wünschenswert, welcher allerdings durch die Veresterung von kommerzieller Polyasparaginsäure, welche auch Verzweigungen enthält, nicht erreicht werden kann. Zudem ist die Umsetzung von Polyasparaginsäure mit Fettalkoholen schwierig durchführbar und erfordert i.A. den ökologisch und ökonomisch unerwünschten Einsatz von organischen Lösungsmitteln.

Diese vorgenannten Probleme lassen sich z.B. durch ein Herstellverfahren lösen, das dadurch gekennzeichnet ist, daß man ein Gemisch von Monoestern monoethylenisch ungesättigter Dicarbonsäuren mit 0.5-1.5 Equivalenten Ammoniak umsetzt bzw. die Ammoniumsalze dieser Säuren thermisch in das Polymer überführt. Die Verwendung von Maleinsäuremonoestern mit nur einer freien Carboxylgruppe gewährleistet dabei den überwiegend linearen Aufbau des resultierenden Polymers. Eingesetzt werden können beispielsweise Derivate der Maleinsäure, Fumarsäure, Itaconsäure, Alkenylbernsteinsäure, Alkylmaleinsäure, Citraconsäure oder deren Ammoniumsalze, vorzugsweise Derivate der Maleinsäure, Fumarsäure oder Itaconsäure, vorzugsweise die Monoester, gegebenenfalls in Gegenwart von Diestern und/oder Anhydriden der genannten Dicarbonsäuren, besonders vorzugsweise Maleinsäurederivate der allgemeinen Formeln (III) und (IV) 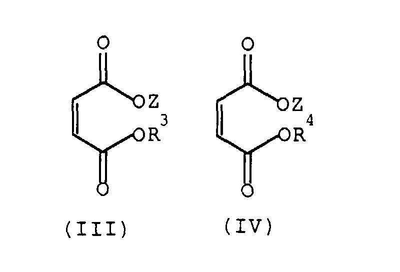 wobei Z für Wasserstoff oder Ammonium, R³ und R⁴ für die oben genannten Reste stehen. Diese Maleinsäurederivate können jeweils alleine oder im Gemisch miteinander eingesetzt werden.

Vorzugsweise eingesetzte Reste R³ sind Alkylreste mit 8 bis 18 C-Atomen, beispielsweise lineare oder verzweigte Decyl-, Dodecyl-, Tetradecyl-, Hexadecyl-, oder Octadecylreste sowie ungesättigte Alkylreste, wie z. B. Oleyl. Vorzugsweise eingesetzte Reste R⁴ sind Alkylreste mit 1 bis 4 C-Atomen, vorzugsweise Methyl, Ethyl, n-Propyl, i-Propyl, n-Butyl, i-Butyl, s-Butyl.

Die Reaktion kann mit oder ohne Zusatz von organischen Lösungsmitteln erfolgen. Als Lösungsmittel kommen beispielsweise Alkohole, Ketone, Ester, Oligo- und Poly(alkylen)glykole bzw. -glykolether, Dimethylsulfoxid, Dimethylformamid, N,N-Dimethylacetamid oder N-Methylpyrrolidon sowie deren Gemische und andere in Frage. Bevorzugt eingesetzt werden Alkohole mit 2-4 C-Atomen sowie Ketone wie z.B. Methylisobutylketon oder Methylisoamylketon oder Alkylester von Carbonsäuren mit 1-4 C-Atomen, wie beispielsweise Essigsäure-sec-Butylester oder Essigsäurepentylester. Die Reaktion kann gegebenenfalls in Gegenwart von verträglichkeitsfördernden Agenzien durchgeführt werden. Dieses können grenzflächenaktive Verbindungen sein, beispielsweise Anlagerungsprodukte von 1 bis 30 Mol Ethylenoxid und/oder 0 bis 5 Mol Propylenoxid an C₁₂₋C₃₀₋Fettalkohole und Wollwachsalkohole; Ethylenoxidanlagerungsprodukte von Glycerinmono- und -diestern und Sorbitanmono- und -diestern von gesättigten und ungesättigten Fettsäuren mit 6 bis 22 C-Atomen; Anlagerungsprodukte von 2 bis 30 Mol Ethylenoxid und/oder 0 bis 5 Mol Propylenoxid an Fettsäuren mit 12 bis 22 C-Atomen und an Alkylphenole mit 8 bis 15 C-Atomen in der Alkylgruppe; C₁₂₋C₁₈₋Fettsäurepartialester von Anlagerungsprodukten von 1 bis 30 Mol Ethylenoxid an Glycerin; Anlagerungsprodukte von Ethylenoxid an Fette und Öle, beispielsweise Rizinusöl oder gehärtetes Rizinusöl; Partialester von gesättigten oder ungesättigten C₁₂₋C₂₂₋Fettsäuren, auch verzweigte oder hydroxysubstituierte, mit Polyolen, beispielsweise Ester von Glycerin, Ethylenglykol, Polyalkylenglykolen, Pentaerythrit, Polyglycerin, Zuckeralkoholen wie Sorbit und Polyglucosiden wie Cellulose; Polysiloxan-Polyalkyl-Polyether-Copolymere und deren Derivate sowie hydrophob modifizierte Polyasparaginsäurederivate, beispielsweise teilveresterte Polyasparaginsäuren, teilveresterte Polyasparaginsäure-co-Glutaminsäuren oder Kondensate aus Maleinsäuremonoestern und Ammoniak, beispielsweise hergestellt nach dem erfindungsgemäßen Verfahren oder nach DE 195 45 678 A, wobei das Herstellungsverfahren der genannten Polyaminosäurederivate keinen Einfluß auf deren verträglichkeitsvermittelnde Wirkung hat. Gegebenenfalls kann auch ein gewisser Teil der Produktmischung im Reaktor verbleiben und als Lösungsvermittler für eine folgende Umsetzung dienen.

Als verträglichkeits- bzw. löslichkeitsvermittelnde Agenzien können auch kationische Tenside, beispielsweise aus der Gruppe der quartären Ammoniumverbindungen, quaternisierten Proteinhydrolysate, Alkylamidoamine, quartären Esterderivate, quartären Silikone, quartären Zucker- und Polysaccharidderivate, und Mischungen daraus, anionische Tenside, beispielsweise aus der Gruppe der Sulfate, Sulfonate, Carboxylate sowie Mischungen derselben, beispielsweise Alkylbenzolsulfonate, α-Olefinsulfonate, α-sulfonierte Fettsäureester, Fettsäureglycerinestersulfate, Paraffinsulfonate, Alkylsulfate, Alkylpolyethersulfate, Sulfobernsteinsäurealkylester, Fettsäuresalze (Seifen), Fettsäureester der Polymilchsäure, N-Acylaminosäureester, N-Acyltaurate, Acylisethionate, Ethercarboxylate, Monoalkylphosphate, N-Acylaminosäurederivate wie N-Acylasparatate oder N-Acylglutamate, N-Acylsarcosinate, amphotere oder zwitterionische Tenside wie beispielsweise Alkylbetaine, Alkylamidoalkylbetaine des Typs Cocoamidopropylbetain, Sulfobetaine, Phosphobetaine, Sultaine und Amidosultaine, Imidazoliniumderivate, Amphoglycinate, oder nichtionische Tenside wie beispielsweise oxethylierte Fettalkohole, oxethylierte Alkylphenole, oxethylierte Fettsäureester, oxethylierte Mono-, Di- oder Triglyceride oder Polyalkylenglykolfettsäureester, Zuckerester, beispielsweise Fettsäureester oder Saccharose, Fructose oder des Methylglucosids, Sorbitolfettsäureester und Sorbitanfettsäureester (gegebenenfalls oxethyliert), Alkyl- oder Alkenylpolyglucoside und deren Ethoxylate, Fettsäure-N-alkylpolyhydroxyalkylamide, Polyglycerinester, Fettsäurealkanolamide, langkettige tertiäre Aminoxide oder Phosphinoxide sowie Dialkylsulfoxide enthalten sein.

Vorzugsweise verbleiben die verträglichkeitsfördernden Agenzien im Produkt. Die Umsetzung zum Copolymeren erfolgt mit wässrigem oder gasförmigen Ammoniak bei Temperaturen von 20 bis 150 °C, sowie nachfolgender Behandlung bei 70 bis 220 °C,vorzugsweise 100 bis 140 °C, unter vermindertem Druck, beispielsweise in Knetapparaturen, Hochviskosreaktoren, Extrudern oder Rührreaktoren, gegebenenfalls unter Einsatz scherkraftreicher Rührer wie Mig- oder Intermig-Rührer.

Unter den Reaktionsbedingungen werden gleichzeitig ein Teil der Estergruppen, bevorzugt die von R⁴OH abgeleiteten, hydrolysiert und die gewünschten Carbonsäure- bzw. Carboxylatgruppen freigesetzt. Durch nachfolgende milde partielle oder vollständige Hydrolyse, bevorzugt der vom kurzkettigen Alkohol R⁴ abgeleiteten Esterfunktionen kann, wenn gewünscht, der Anteil freier Säuregruppen weiter erhöht werden, beispielsweise durch Umsetzung mit Wasser ggf. in Gegenwart von Säuren oder Basen, oder mit Alkalimetallhydroxiden.

Die eingesetzten Polymeren können nachbehandelt werden, beispielsweise durch Behandlung mit Aktivkohle, Bleichung mit Oxidationsmitteln wie H₂O₂, Cl₂, Ozon, Natriumchlorit, Natriumhypochlorit etc. oder Reduktionsmitteln wie z. B. NaBH, oder H₂ in Gegenwart von Katalysatoren.

Ein alternativer Syntheseweg, der über die Ringöffnung von nach üblichen Verfahren hergestellten Polysuccinimid mit Fettalkoholen oder die Umsetzung von Polysuccinimid mit kurzkettigen Alkoholen sowie die nachträgliche Umesterung der Produkte mit Fettalkoholen und abschliessende Hydrolyse verläuft, führt nicht zu den gewünschten verzweigungsfreien copolymeren Polyasparaginsäureestern.

Der Anteil der Polyasparaginsäurederivate in den erfindungsgemäßen tensidischen Zubereitungen kann 0.1 bis 40 Gew.-%, vorzugsweise 1 bis 15 Gew.-% betragen, wobei der Gesamtanteil aller mit diesen in Kombination eingesetzten Tenside 1 bis 95 Gew.-%, vorzugsweise 1 bis 50 Gew.-% beträgt. Es sind feste, flüssige oder pastöse Zubereitungen möglich, z.B. Seifenstücke, Waschlotionen, Duschgele, Shampoos.

Die biologisch abbaubaren Polyasparaginsäurederivate mit einem naturnahen Polyaminosäurerückgrat sind ausgesprochen milde Tenside, welche in Kombination mit anionischen, kationischen, nichtionischen, zwitterionischen und/oder amphoteren Tensiden eine überraschende Mildheit besitzen und hervorragendes Schaumverhalten zeigen. Sie sind schäumend und stabilisieren andere tensidische Schäume. Mit Hilfe der hier beschriebenen Polyasparaginsäurederivate ist ein Zugang zu milden Tensidformulierungen möglich, ohne Einbußen oder Nachteile bezüglich Reinigungswirkung und Schaumverhalten in Kauf nehmen zu müssen. Durch die Auswahl der geeigneten Polyasparaginsäurederivate können die gewünschten Eigenschaften der Tensidformulierung (z.B. Schaum schwach, reich oder cremig, Viskosität, Betonung der pflegenden oder reinigenden Wirkung) erhalten bleiben oder sogar gefördert werden.

Die erfindungsgemäß in Kombination mit Polyasparaginsäurederivaten eingesetzten Tenside können beispielsweise anionische Tenside aus der Gruppe der Sulfate, Sulfonate, Carboxylate sowie Mischungen derselben sein. Die anionischen Gruppen können in neutralisierter Form vorliegen, mit kationische Gegenionen aus der Gruppe der Alkalimetalle, z.B. Natrium oder Kalium, Erdalkalimetalle (z.B. Magnesium oder Calcium), Ammonium oder substituiertem Ammonium, z.B. Tetraalkylammonium oder Mono/Di/Triethanolammonium. Eingesetzt werden beispielsweise Alkylbenzolsulfonate, α-Olefinsulfonate, α-sulfonierte Fettsäureester, Fettsäureglycerinestersulfate, Paraffinsulfonate, Alkylsulfate, Alkylpolyethersulfate, Sulfobernsteinsäurealkylester, Fettsäuresalze (Seifen), Fettsäureester der Polymilchsäure, N-Acylaminosäureester, N-Acyltaurate, Acylisethionate, Ethercarboxylate, Monoalkylphosphate, N-Acylaminosäurederivate wie N-Acylaspartate oder N-Acylglutamate, N-Acylsarcosinate und andere. Der Anteil an anionischen Tensiden in den erfindungsgemäßen Tensidzubereitungen kann zwischen 0 und 95 Gew.-% betragen.

Die erfindungsgemäß in Kombination mit Polyasparaginsäurederivaten eingesetzten Tenside können beispielsweise kationische Tenside, beispielsweise aus der Gruppe der quartären Ammoniumverbindungen, quaternisierten Proteinhydrolysate, Alkylamidoamine, quartären Esterderivate, quartären Silikone, quartären Zucker- und Polysaccharidderivate, und Mischungen daraus, sein.

Die erfindungsgemäß in Kombination mit Polyasparaginsäurederivaten eingesetzten Tenside können beispielsweise amphotere oder zwitterionische Tenside sein, welche sowohl anionische Gruppen (wie z.B. Carboxylat oder Sulfonat sowie Mischungen daraus) als auch entweder Amingruppen (bei amphoteren Tensiden) oder (bei zwitterionischen Tensiden) kationische Gruppen wie Ammonium, Phosphonium oder Sulfonium sowie Mischungen daraus tragen. Zu dieser Gruppe gehören beispielsweise Alkylbetaine, Alkylamidoalkylbetaine des Typs Cocoamidopropylbetain, Sulfobetaine, Phosphobetaine, Sultaine und Amidosultaine, Imidazoliniumderivate, Amphoglycinate, polyfunktionelle amphotere Tenside (Lomax-Typen). Der Anteil an amphoteren und/oder zwitterionischen Tensiden in den erfindungsgemäßen Tensidzubereitungen kann zwischen 0 und 95 Gew.-% betragen.

Die erfindungsgemäß in Kombination mit Polyasparaginsäurederivaten eingesetzten Tenside können beispielsweise nichtionische Tenside sein, welche beispielsweise aus der Verknüpfung von Alkylenoxidgruppen hydrophiler Natur, vorzugsweise auf der Basis von Ethylenoxid oder Ethylenoxid/Propylenoxid, mit hydrophoben Alkyl oder Arylresten hervorgehen. Beispiele sind oxethylierte Fettalkohole, oxethylierte Alkylphenole, oxethylierte Fettsäureester, oxethylierte Mono-, Di- oder Triglyceride oder Polyalkylenglykolfettsäureester. Andere nichtionische Tenside können aus der Gruppe der Alkylpolysaccharide, z.B. Alkylpolyglucoside, Zuckerester, z.B. Fettsäureester der Glucose, Saccharose, Fructose oder des Methylglucosids, Sorbitolfettsäureester und Sorbitanfettsäureester (ggf. oxethyliert), Polyglycerinester, Fettsäurealkanolamide, N-Acylaminozuckerderivate z.B. N-Acylglucamine, langkettige tertiäre Aminoxide oder Phosphinoxide sowie Dialkylsulfoxide stammen. Die angegebenen Tensidtypen können alleine oder in Mischungen eingesetzt werden. Der Gehalt an nichtionischen Tensiden in den erfindungsgemäßen Tensidzübereitungen kann zwischen 0 und 95 Gew.-% betragen.

In vorteilhafter Weise umfassen die Tensidzubereitungen neben wenigstens einem Polyasparaginsäurederivat wenigstens ein weiteres Tensid aus der Gruppe der nichtionischen, kationischen, zwitterionischen oder amphoteren Tenside, oder zwei anionische Tenside oder wenigstens ein anionisches Tensid in Kombination mit wenigstens einem weiteren Tensid aus der Gruppe der anionischen, nichtionischen, kationischen, zwitterionischen oder amphoteren Tenside, oder wenigstens ein nichtionisches Tensid sowie wenigstens ein weiteres Tensid aus der Gruppe der anionischen, nichtionischen, kationischen, zwitterionischen oder amphoteren Tenside. Auch sind Tensidzubereitungen, welche wenigstens ein amphoteres oder zwitterionisches Tensid, wenigstens ein Polyasparaginsäurederivat sowie wenigstens ein weiteres Tensid aus der Gruppe der anionischen, nichtionischen, kationischen, zwitterionischen oder amphoteren Tenside enthalten, in vorteilhafter Weise einsetzbar. In diesen Fällen beträgt die Konzentration der Polyasparaginsäurederivate 0.1 bis 40 Gew.-%, insbesondere 1 bis 15 Gew.-%, die der weiteren Tensidkomponenten jeweils 1 bis 80 Gew.-%, vorzugsweise 1 bis 50 Gew.-%, bei flüssigen Zubereitungen vorzugsweise 1 bis 30 Gew.-%.

Die erfindungsgemäßen Tensidzubereitungen können weitere Hilfs- und Zusatzstoffe enthalten wie beispielsweise:
Wasser, Lösungsmittel z.B. aus der Gruppe der Alkohole, vorzugsweise der aliphatischen Alkohole, und Polyole wie z.B. Ethanol, Propanol, i-Propanol, Propylenglykol, Glycerin oder Polyethylenglykole, sowie Gemische daraus;
Verdickungsmittel, z.B. Kaolin, Bentonit, Fettsäuren, höhere Fettalkohole, Stärke, Polyacrylsäure und deren Derivate, Cellulosederivate, Alginate;
Trübungsmittel, z.B. Glykolesterderivate;
Moisturizer, z.B. langkettige Fettsäuren oder deren Ester, flüssige, wasserlösliche Polyole wie Glycerin, Sorbitol, Polyethylenglokol, Propylenglykol, Milchsäure, biogene Aminosäuren, etc.;
Emollients wie tierische und pflanzliche Öle, Carbonsäureester, Lanolin, Bienenwachs, Silicone;
Polymere Agenzien zur Verbesserung des Hautgefühls wie z.B. anderem nonionic guar gums, nichtionische Cellulosederivate wie Hydroxyethylcellulose und Carboxymethylcellulose;
konditionierende, pflegende oder pharmazeutisch wirksame Bestandteile wie z.B. kationische oder amphotere Polymere, Proteine und Proteinderivate, Lanolinderivate, Panthothensäure, Betain, Polydimethylsiloxane oder deren Derivate, Sonnenschutzwirkstoffe wie z.B. p-Aminobenzoesäure und deren Derivate, Stilbenderivate etc.; sowie
Lösungsvermittler, Stabilisatoren, Puffersubstanzen, Geruchsstoffe, Konservierungsmittel und/oder Farbstoffe.

Die Polyasparaginsäurederivate enthaltenden Tensidzubereitungen lassen sich vorteilhaft anwenden in z.B. Haarshampoos, Duschbädern, Schaumbadzubereitungen, Hand-, Gesichts- und Intimreinigungslotionen, Flüssigseifen, Seifenstücke, Rasiercremes, Handwaschpasten, hautfreundlichen Geschirrspülmitteln, Reinigungsmitteln für glatte Oberflächen sowie in Zahncremes. Sie ergeben einen reichen, cremigen Schaum, sind leicht auswaschbar und zeichnen sich durch ihre Milde und ihr angenehmes Hautgefühl aus. Die Mildheit der Tensidkompositionen lässt sich beispielsweise im RBC-Test nach Pape und Hoppe (Drug Res. 1990, 40, 498-502) nachweisen, wobei die schädigende Wirkung auf die roten Blutkörperchen mit der z.B. im Draize-Test erfassbaren schleimhautreizenden Wirkung korreliert.

### Beispiele:

### Beispiele 1 bis 5

### Poly(asparaginsäure-co-alkylaspartat)

Die Herstellung der Polyasparaginsäureester erfolgte in Analogie zu DE 195 45 678 A durch Umsetzen der Edukte (Monoethylmaleat, Monoalkylmaleat) in Methylisobutylketon mit 1.0 bis 1.5 Äquivalenten an Ammoniakgas und Erhitzen i. Vak. auf 120-140 °C für 4-6 h.

| Beispiel | Alkylrest | Edukt: mol Alkylmaleat | Edukt: mol Ethylmeleat | Produkt: mol% Alkylester | Produkt: mol% Ethylester | mol% Säure |
|---|---|---|---|---|---|---|
| 1 | Decyl | 1.0 | 3.0 | 20 | 5 | 70 |
| 2 | Decyl | 1.4 | 2.6 | 30 | 2 | 68 |
| 3 | Dodecyl | 1.2 | 2.8 | 27 | 5 | 68 |
| 4 | Cetyl | 1.0 | 3.0 | 25 | 5 | 70 |
| 5 | Stearyl | 0.8 | 3.2 | 20 | 7 | 73 |

### Vergleichs-Beispiele 1 bis 3

11.5 g Polyasparaginsäure (durch Behandlung des Natriumsalzes mit einem sauren Ionenaustauscher, Mw = 6000 nach GPC mit Eichung gegen Polyacrylat) wurden unter den folgenden Bedingungen mit 0.5-5 Äquivalenten Fettalkohol pro Asparaginsäureeinheit zur Reaktion gebracht:

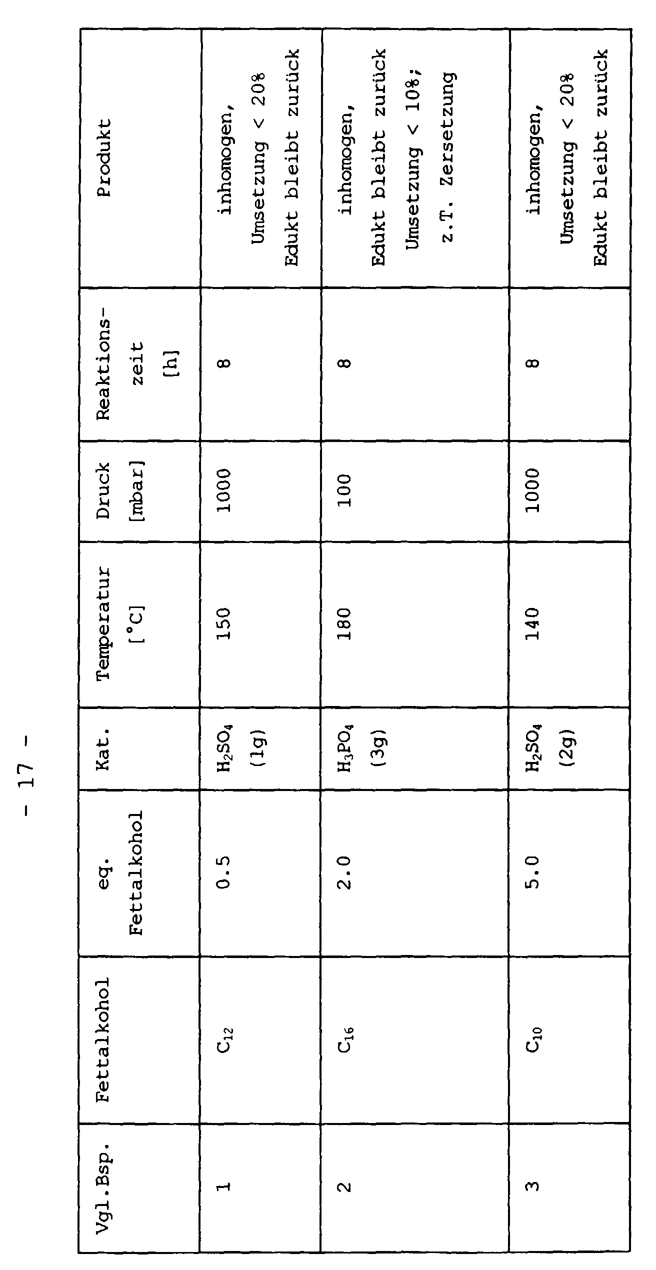

### Beispiele 6 bis 12, Vergleichsbeispiele 4 bis 8

### Einfache Tensidzubereitungen mit Polyasparaginsäuretensiden verwendete Tenside:

- LES:: Natriumlaurylethersulfat, z.B. Texapon® N28, Henkel
- BET:: Cocoamidopropylbetain, z.B. TEGO® Betain F50, Th. Goldschmidt
- GLUC:: Alkylpolyglucosid, z.B. TEGO® Glucosid 1216, Th. Goldschmidt
- GLYC:: Natriumcocoamphocarboxylglycinat, z.B. TEGO® Glycinat 818, Th. Goldschmidt
- SULF:: Natriumlaurylethersulfosuccinat,z.B.TEGO® Sulfosuccinat F30, Th. Goldschmidt
- OLEF:: Natrium-C14/16-Olefinsulfonat, z.B. Elfan® OS46, AKZO
- SORB:: PEG-80-Sorbitanlaurat, z.B. ATLAS® G4280, ICI
- PA-10:: Poly(asparaginsäure-co-decylaspartat) nach Bsp.1, (48%ig in Wasser, pH 5.5)
- PA-12:: Poly(asparaginsäure-co-laurylaspartat) nach Bsp 3, (25%ig in Wasser, pH 5.5)

Die Bestimmung der Schleimhautverträglichkeit erfolgte durch den RBC-Test gemäß Pape und Hoppe (Drug Res. 1990, 40, 498-502). Zur Messung wurden die Tensidbasismischungen (Gesamttensidgehalt an Primär-, Sekundär- und Polyasparaginsäuretensid = 25 Gew.-%) auf die Testkonzentration von 0.1 % (für die Bestimmung des H50-Wertes) bzw. 1.0 % (für die Bestimmung des DI-Wertes) verdünnt.

| Bsp. | Tensid A | Tensid B | Tensid A/B | Polyaspartat | Anteil | H50 [mg/l] | DI/% | L/D |
|---|---|---|---|---|---|---|---|---|
| 6 | LES | | | PA-10 | 25% | 30 | 28 | 1.07 |
| Vgl. 4 | LES | BET | 3:1 | PA-10 | 0 | 19 | 27 | 0.70 |
| 7 | LES | BET | 3:1 | PA-10 | 14.5% | 24 | 25 | 0.96 |
| 8 | LES | BET | 3:1 | PA-12 | 15% | 25 | 24 | 1.0 |
| Vgl. 5 | LES | GLUC | 3:1 | PA-10 | 0 | 26 | 30 | 0.87 |
| 9 | LES | GLUC | 3:1 | PA-10 | 14.5% | 35 | 29 | 1.2 |
| Vgl. 6 | LES | GLYC | 3:1 | PA-10 | 0 | 22 | 28 | 0.79 |
| 10 | LES | GLYC | 3:1 | PA-10 | 14.5% | 26 | 19 | 1.4 |
| Vgl. 7 | SULF | BET | 3:1 | PA-10 | 0 | 39 | 34 | 1.1 |
| 11 | SULF | BET | 3:1 | PA-10 | 14.5% | 50 | 23 | 2.2 |
| Vgl. 8 | OLEF | SORB | 3:1 | PA-10 | 0 | 19 | 49 | 0.39 |
| 12 | OLEF | SORB | 3:1 | PA-10 | 14.5 | 24 | 40 | 0.60 |

Die erfindungsgemäßen Beispiele demonstrieren die milderen Eigenschaften von verschiedenen erfindungsgemäßen Tensidformulierungen mit Polyasparaginsäurederivaten.

### Beispiel 13

### Verbesserung der Schaumeigenschaften von Tensiden durch Polyasparaginsäurederivate:

| Rezeptur | (A) | (B) |
|---|---|---|
| Poly(asparaginsäure-co-decylaspartat) nach Bsp.1, (48%ig in Wasser, pH 5.5) | 0.0% | 1.0% |
| Texapon® N28 (28% Natriumlaurylethersulfat, Henkel) | 21.4% | 21.4% |
| Tego® Betain F50 (37.5% Cocoamidopropylbetain, Th. Goldschmidt) | 16.0% | 16.0% |
| Wasser ad 100%, pH ad 6.0 | | |

Die Schaumeigenschaften der Tensidmischung werden durch Aufschäumen einer verdünnten Tensidlösung bestimmt.(0.5 Gew.-% WAS, 8 °dH, 30 °C, Ystral-Leitstrahlmischer, 750 W, 2 min)

| Mischung | Schaumvolumen [ml] | Wasserseparation 10 min [ml] | Schaumdichte [g/ml] |
|---|---|---|---|
| A | 1490 ± 17 | 240 ± 2.0 | 0.208 ± 0.002 |
| B | 1593 ± 12 | 233 ± 2.9 | 0.196 ± 0.003 |

Dieses Beispiel belegt den positiven Einfluß von Polyasparaginsäurederivaten auf das Schaumverhalten von Tensidsystemen.

### Beispiel 14

### Formulierung für ein Duschgelkonzentrat

| | |
|---|---|
| Poly(asparaginsäure-co-decylaspartat),nach Bsp. 1, (48%ig in Wasser, pH 5.5) | 9.0 % |
| Texapon® N70 (70% Natriumlaurylethersulfat, Henkel) | 32.0 % |
| Tagat® R40 (PEG-40-Ethoxylat von hydriertem Rizinusöl, Th. Goldschmidt) | 5.0 % |
| Tego® Glucosid 810 (60% Capryl/Capringlucosid, Th. Goldschmidt) | 9.0 % |
| Zitronensäure (20%) | 0.9 % |
| NaCl (25%) | 8.5 % |
| Wasser | 17.6 % |
| Tego® Betain F50 (37.5% Cocoamidopropylbetain, Th. Goldschmidt) | 18.0 % |

Ergebnisse des RBC-Tests: H50 = 30 mg/l , DI=30 %, L/D= 1.6

### Beispiel 15

### Hand- und Gesichtsreinigungsformulierung für Babys:

| | |
|---|---|
| Poly(asparaginsäure-co-cetylaspartat), nach Bsp. 4, (25%ig in Wasser, pH 5.5) | 3.0 % |
| Tagat® R40 (PEG-40-Ethoxylat aus hydriertem Rizinusöl,Th. Goldschmidt) | 2.0 % |
| Poly(asparaginsäure-co-decylaspartat), nach Bsp. 2, (25%ig in Wasser, pH 5.5) | 6.0 % |
| Tegosoft® LSE 65K (Saccharosecocoat, Th. Goldschmidt) | 2.0 % |
| Tegosoft® GC (PEG-7 Glycerylcocoat, Th. Goldschmidt) | 1.5 % |
| Texapon® N28 (28% Natriumlaurylethersulfat, Henkel) | 23.0 % |
| Parfümöl | 0.2 % |
| Wasser | 52.3 % |
| Tego® Betain F50 (37.5% Cocoamidopropylbetain, Th. Goldschmidt) | 5.0 % |
| Antil® 171 (PEG-18 Glyceryloleat/cocoat, Th. Goldschmidt) | 3.0 % |
| Tego® Pearl N100 (Ethylenglycoldistearat, Stearylethoxylat,Th. Goldschmidt) | 2.0 % |
| pH ad 6.0 | |

### Beispiel 16

### Flüssigseifenformulierung:

| | |
|---|---|
| Poly(asparaginsäure-co-N-decylaspartamid) nach Bsp. 6 (25%ig in Wasser, pH 6.0) | 12.0 % |
| Texapon® N28 (28% Natriumlaurylethersulfat, Henkel) | 30 % |
| Parfümöl | 0.5 % |
| Abil® B8851 (Siliconpolyether, Th. Goldschmidt) | 0.5 % |
| Wasser | 50.0 % |
| Tego® Betain F50 (37.5% Cocoamidopropylbetain, Th. Goldschmidt) | 2.0 % |
| Antil® 171 (PEG-18 Glyceryloleat/cocoat, Th. Goldschmidt) | 5.0 % |

### Beispiel 17

### PEG-freie Duschgelformulierung:

| | |
|---|---|
| Poly(asparaginsäure-co-laurylaspartat), nach Bsp. 3, (30%ig in Wasser, pH 5.5) | 22.0 % |
| Elfan® OS46 (37% Natrium-C14/16-Olefinsulfonat, AKZO) | 20.0 % |
| Parfümöl | 0.5 % |
| Wasser | 46.5 % |
| Lactil® (Natriumlactatzübereitung, Th. Goldschmidt) | 1.0 % |
| Tego® Betain F50 (37.5% Cocoamidopropylbetain, Th. Goldschmidt) | 8.0 % |
| Antil® HS60 (Cocoamidopropylbetain, Glyceryllaurat, Th. Goldschmidt) | 2.0 % |
| pH ad 5.5 | |

### Beispiel 18

### Seifenstück mit Polyasparaginsäuretensiden

| | |
|---|---|
| Grundmasse | 96.0 % |
| Weizenstärke | 2.0 % |
| Tegosoft® OL (Octyllaurat, Th. Goldschmidt) | 1.0 % |
| Natriumsulfat | 1.0 % |

| (Grundmasse) | |
|---|---|
| Tego® Sulfosuccinat F 30 (30% Natriumlaurylethersulfosuccinat, Th.Goldschmidt) | 5.0 % |
| Poly(asparaginsäure-co-stearylaspartat), nach Bsp.5, (50%ig in Wasser, pH 6.0) | 2.5 % |
| Poly(asparaginsäure-co-decylaspartat), nach Bsp. 2, (50%ig in Wasser, pH 6.0) | 7.5 % |
| Elfan® AT 84 (Isethionat, AKZO) | 36.0 % |
| Tego® Glycinate 818 D (Dinatriumcocoamphodiacetat, Th. Goldschmidt) | 3.0 % |
| Tego® Betain CKD (80% Cocoamidopropylbetain, Th. Goldschmidt) | 8.5 % |
| Te® Cerowachs 1030 K (Th. C. Tromm) | 10.0 % |
| Te® hell 6403 Paraffin (Schümann/Sasol) | 11.5 % |
| Tego® Alkanol 1618 (Cetearylalkohol, Th. Goldschmidt) | 6.5 % |
| Tegin® M (Glycerylstearat, Th. Goldschmidt) | 9.0 % |
| Datamuls® 43 (Diacetylweinsäureester von Fettsäureglyceriden, Th. Goldschmidt) | 0.5 % |

### Beispiel 19

### Phosphathaltige Zahncreme

| | |
|---|---|
| Poly(asparaginsäure-co-laurylaspartat), nach Bsp. 3, (25%ig in Wasser, pH 5.5) | 5.0 % |
| Wasser | 34.7 % |
| Natriumbenzoat | 0.2 % |
| Natrosol® 250HR (Hydroxyethylcellulose) | 1.8 % |
| Xylit | 0.3 % |
| Sorbitol (70%) | 12.0 % |
| Tego® Betain CKD (Cocoamidopropylbetain, Th. Goldschmidt) | 1.5 % |
| Abil® B8851 (Siliconpolyether, Th. Goldschmidt) | 2.0 % |
| Natriumfluorid | 0.2 % |
| Dentphos® K (Calciumphosphat) | 33.0 % |
| Sident® 12 (Siliciumdioxid) | 5.0 % |
| Aerosil® 200 (Siliciumdioxid) | 3.0 % |
| Titandioxid mikro | 0.2 % |
| Tagat® S (PEG-30 glycerylstearat, Th. Goldschmidt) | 0.5 % |
| Aromaöl | 0.6 % |

## Patentansprüche

1. Tensidische Zubereitungen für Reinigungsmittel und/oder kosmetische Mittel, enthaltend ein oder mehrere überwiegend geradkettige copolymere Polyasparaginsäurederivate, die von den Ammoniumsalzen von Monoestern α,β-ungesättigter Dicarbonsäuren oder den Monoestern der genannten Dicarbonsäuren und Ammoniak abgeleitet sind, gegebenenfalls hergestellt in Gegenwart von deren Diestern und/oder Anhydriden und wenigstens ein weiteres Tensid neben Polyasparaginsäurederivaten aus der Gruppe der nichtionischen, kationischen, amphoteren und zwitterionischen Tenside sowie deren Mischungen oder wenigstens ein anionisches Tensid in Kombination mit wenigstens einem anionischen, kationischen, nichtionischen, zwitterionischen und/oder amphoteren Tensid, neben üblichen Hilfs- und Zusatzstoffen.

2. Tensidzubereitungen nach Anspruch 1, enthaltend neben Polyasparaginsäurederivaten wenigstens ein nichtionisches Tensid neben wenigstens einem oder mehreren weiteren Tensiden aus der Gruppe der anionischen, kationischen, nichtionischen, zwitterionischen und/oder amphoteren Tenside.

3. Tensidzubereitungen nach Anspruch 1, enthaltend neben Polyasparaginsäurederivaten wenigstens ein amphoteres oder zwitterionisches Tensid neben wenigstens einem oder mehreren weiteren Tensiden aus der Gruppe der anionischen und/oder kationischen und/oder nichtionischen, zwitterionischen und/oder amphoteren Tenside.

4. Tensidzubereitungen nach Anspruch 1, enthaltend neben Polyasparaginsäurederivaten wenigstens ein weiteres Tensid aus der Gruppe der nichtionischen, kationischen, amphoteren und zwitterionischen Tenside.

5. Tensidzubereitungen nach einem oder mehreren der Ansprüche 1 bis 4, dadurch gekennzeichnet, daß die α,β-ungesättigten Carbonsäuren Maleinsäure und/oder Fumarsäure sind.

6. Verfahren zur Herstellung der Polyasparginsäurederivate nach Anspruch 1 dadurch gekennzeichnet, daß man Ester α,β-ungesättigter Dicarbonsäuren oder deren Ammoniumsalze, insbesondere Maleinsäurederivate der allgemeinen Formel 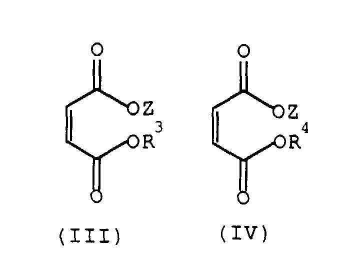 allein oder im Gemisch miteinander mit Ammoniak umsetzt und polymerisiert wobei
Z für Wasserstoff oder Ammonium,
R³ für gleiche oder verschiedene, geradkettige oder verzweigte, gesättigte oder ungesättigte Alkylreste R⁹ mit 6 bis 24 C-Atomen oder Radikale der Struktur -X-R⁹, wobei X eine Oligo- oder Polyoxyalkylenkette mit 1 bis 100 Oxyalkyleneinheiten ist, und
R⁴ für gleiche oder verschiedene, geradkettige oder verzweigte, gesättigte oder ungesattigte Alkylreste mit 1 bis 5 C-Atomen steht,
gegebenenfalls in Gegenwart von bis zu 20 Gew.-% proteinogene oder nichtproteinogene Aminosäuren oder deren Derivate sowie gegebenenfalls in weiteren Schritten durch Hydrolyse zu Gruppen der Formel I 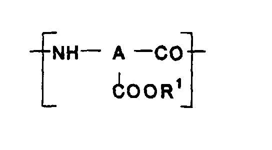 wobei R¹ die Bedeutung von Alkali-, Erdalkalimetall, Wasserstoff, Ammonium oder alkylsubstituiertem Ammonium, [NR⁵R⁶R⁷R⁸]+, worin R⁵, R⁶, R⁷ und R⁸ unabhängig voneinander für Wasserstoff, Alkyl oder Hydroxyalkyl mit 1 bis 30 C-Atomen steht, hat,
unter Verwendung von Estern oder Ketonen als Lösungsmittel umsetzt.

7. Verfahren zur Herstellung der Polyasparaginsäurederivate nach Anspruch 1, dadurch gekennzeichnet, daß man die Reaktion in Gegenwart von verträglichkeitsfördernden Agenzien, insbesondere aus der Gruppe der anionischen, nichtionischen, zwitterionischen, kationischen und amphoteren Tensiden, sowie aus der Gruppe der hydrophob modifizierten Polyasparaginsäurederivate, durchführt.

8. Tensidzubereitungen nach einem oder mehreren der Ansprüche 1 bis 7, enthaltend 0,1 bis 40 Gew.-%, insbesondere 1 bis 15 Gew.-% Polyasparaginsäurederivate bei einer Gesamttensidkonzentration von 1 bis 95 Gew.-%, insbesondere 1 bis 50 Gew.-%.

9. Verwendung der Tensidzubereitungen nach einem oder mehreren der Ansprüche 1 bis 8 in milden, schäumenden Tensidsystemen für Shampoos, Waschlotionen und Reinigungsmittel für Gesicht, Haar, Haut und Intimbereich, Rasiercreme und -lotionen, Flüssigseifen, Geschirrspülmitteln, Reinigungsmitteln für glatte Oberflächen, Seifenstücken, Schaumbädern, Duschgelen sowie in Zahncremes und/oder Mundspülungen.
